(19) Europäisches Patentamt
European Patent Office
Office européen des brevets

(11) Veröffentlichungsnummer: **0 399 409 A2**

(12) # EUROPÄISCHE PATENTANMELDUNG

(21) Anmeldenummer: **90109520.8**

(22) Anmeldetag: **19.05.90**

(51) Int. Cl.5: **C07D 263/56, C07D 277/64, C12Q 1/34, G01N 33/58, C12N 11/00, C07F 9/09**

(30) Priorität: **23.05.89 DE 3916729**

(43) Veröffentlichungstag der Anmeldung: **28.11.90 Patentblatt 90/48**

(84) Benannte Vertragsstaaten: **AT BE CH DE ES FR GB IT LI LU NL SE**

(71) Anmelder: **BEHRINGWERKE Aktiengesellschaft
Postfach 1140
D-3550 Marburg 1(DE)**

(72) Erfinder: **Habenstein, Klaus, Dr.
Im Ketzergrund 33
D-3552 Wetter(DE)**

(74) Vertreter: **Klein, Otto, Dr. et al
Hoechst AG Zentrale Patentabteilung
Postfach 80 03 20
D-6230 Frankfurt am Main 80(DE)**

(54) **Fluorogene Verbindungen und ihre Verwendung.**

(57) Die Erfindung bezieht sich auf fluorogene Verbindungen für den Nachweis hydrolysierender Enzyme und die Verwendung der fluorogenen Verbindungen.

EP 0 399 409 A2

## Fluorogene Verbindungen und ihre Verwendung

Die Erfindung bezieht sich auf fluorogene Verbindungen für den Nachweis hydrolysierender Enzyme und die Verwendung der fluorogenen Verbindungen.

Hydrolysierende Enzyme, die sogenannten Hydrolasen, sind im tierischen Organismus für eine Vielzahl von Reaktionen verantwortlich. Dabei werden Hydrolasen verschiedener Funktion nach ihrer Spezifität unterschieden wie z.B.:

- Esterasen, wie z. B. die Acetylcholinesterase, die Carbonylester hydrolysieren.
- Glykosidasen, wie z. B. die ß-D-Galactosidase, die die o-glykosidischer Verknüpfung von Zucker untereinander oder mit Alkoholen hydrolysieren.
- Phosphatasen, wie z. B. alkalische Phosphatase, die Phosphorsäureester hydrolysieren.
- Sulfatasen, wie z. B. Iduronatsulfatase, die Schwefelsäureester hydrolysieren.

Die Hydrolasen gehören zu den wichtigsten Enzymen des tierischen Organismus. Ihr Fehlen, vermindertes oder vermehrtes Vorkommen deutet häufig auf ernste Erkrankungen des Organismus hin. Ein bekanntes Beispiel ist die Mucopolysaccharidose; diese rezessiv vererbliche Krankheit, für die 7 verschiedene Manifestationsformen unterscheidbar sind, beruht auf einem genetisch determinierten Defekt von Hydrolasen, z. B. der ß-Galaktosidase im Fall der Morbus Morquio und der Iduronatsulfatase bei Morbus Hunter. Morbus Morquio beispielsweise kann durch Bestimmung des ß-D- Galactosidase-Spiegels von Fibroblasten oder Leucozyten eindeutig diagnostiziert werden. Der Spiegel einer anderen Glykosidase, der Amylase, in Blut oder Harn wird zur Diagnose von Pankreaserkrankungen herangezogen. Darüber hinaus finden hydrolytische Enzyme als diagnostische Hilfsmittel, sogenannte Marker, z. B. in Enzymimmunoassays Verwendung.

Die Quantifizierung hydrolysierender Enzyme zu diagnostischen Zwecken setzt hochempfindliche und spezifische Nachweissysteme voraus, um selbst geringe Enzymkonzentrationen exakt bestimmen zu können. Dabei sind die natürlichen Substrate zum Nachweis ungeeignet, da schon vor der Durchführung des Tests Hydrolyseprodukte in den Proben vorhanden sind oder aber die Hydrolyseprodukte sehr schwer bestimmbar sind. Im Stand der Technik werden daher künstliche Substrate verwendet, deren Hydrolyseprodukte physikalisch oder chemisch nachweisbar sind. In der Regel erfolgt der Nachweis durch Bestimmen der in der hydrolytischen Reaktion freigesetzten Mengen an fluoreszierenden oder stark absorbierenden farbigen Substanzen. Letztere haben nun zwar den Vorteil, daß sie auch visuell, d. h., ohne apparative Hilfsmittel wahrgenommen und somit einer direkten Auswertung zugänglich sind. Andererseits ist die selbst mit apparativen Hilfsmitteln erreichbare Sensitivität chromogener Tests nicht immer ausreichend. Für hochempfindliche Nachweise bedient man sich deshalb schon seit langem der Fluoreszenzmeßtechnik, die im allgemeinen eine um den Faktor $10^3$ höhere Signalausbeute und damit Testempfindlichkeit als chromogene Tests erreichen läßt. Insbesondere in Im munoassays werden daher in steigendem Maße die bisherigen Radioisotope als Label ersetzt durch Fluoreszenzlabel (J. Pharm. Biomed. Anal. 5 (7), 1987, 649-58). Hierbei eröffnet insbesondere die enzymverstärkte Fluoreszenzmeßtechnik neue Wege zur Entwicklung hochsensitiver Testverfahren. Wie jedoch in dem o. g. Literaturzitat, Seiten 651 und 652, sowie in Clin. Chem. 25 (3), 1979, 353-355, beschrieben, ergeben sich infolge unzureichender Fluorophore erhebliche Schwierigkeiten bei der allgemeinen Einführung dieses vielversprechenden Meßverfah- rens. So besitzen z. B. Fluoreszenzfarbstoffe von Typ Fluoreszein, Rhodamin oder Resorufin einen außerordentlich kleinen Stokes-Shift (Differenz zwischen Anregungs- und Emissionswellenlängenmaximum) von ca. 20 nm. Fluorophore vom Typ Umbelliferon besitzen zwar einen Stokes-Shift von ca. 70 nm, emittieren jedoch zu kurzwellig (450 nm), so daß die um oder oberhalb von 400 nm liegende Eigenfluoreszenz von Probenbestandteilen sowie von Träger- und Gefäßmaterialien die Meßergebnisse verfälschen. Wieder anderen Fluorophoren fehlt die für o.g. Hydrolasensubstrate essentielle Hydroxylgruppe im Molekül.

Der vorliegenden Erfindung lag daher die Aufgabe zugrunde, fluorogene Verbindungen für den Nachweis hydrolysierender Enzyme zur Verfügung zu stellen, deren Hydrolyse spezifisch und hochempfindlich zu meßbaren Fluoreszenzsignalen mit Maxima um oder oberhalb 500 nm führt. Diese Aufgabe wird erfindungsgemäß dadurch gelost, daß die fluorogene Verbindung eine Verbindung der allgemeinen Formel I ist.

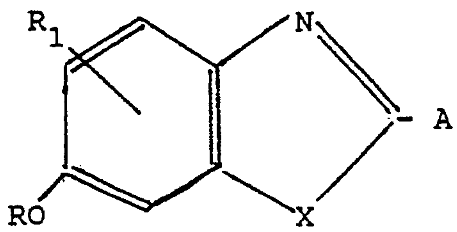

I

wobei
X = O oder S bedeutet
R = ein durch enzymatisch katalysierte Hydrolyse abspaltbarer Rest ist,
$R_1$ = H, $C_1$ bis $C_4$ Alkyl oder Phenyl ist, das mit 1 bis 3 $-CH_3$ substituiert sein kann,
A = eine die Mesomerie des benzoheterocyclischen Ringsystems verlängernde Molekülstruktur aufweist, insbesondere aromatische oder heteroaromatische, auch substituierte und ggf. benzoanellierte 6- und 5-Ringsysteme umfaßt, die auch über eine oder mehrere Mehrfachbindungen in 2-Stellung des Benzoheterocyclus angreifen können, oder eine elektronenziehende Gruppe wie CN oder $CF_3$.

Bevorzugt sind Verbindungen der Formel I, worin
A einen Phenyl- oder Naphtylrest bedeutet, der gegebenenfalls durch 1 bis 3 elektronegative Gruppen, wie z. B. -CN, $-CF_3$, Thiazol oder Benzothiazol substituiert ist
oder
einen Thiazol-, Oxazol-, Benzothiazol- oder Benzoxazolrest, der gegebenenfalls mit 1 bis 3 elektronegativen Gruppen , wie z. B. -CN oder $-CF_3$ substituiert ist, bedeutet.

Besonders bevorzugt sind Verbindungen, worin
A Phenyl oder mit bis zu 3 -CN, $-CF_3$, Thiazol- oder Benzothiazolresten substituiertes Phenyl bedeutet.

Ferner sind bevorzugt Verbindungen, worin
R ein Carbonsäure-, ein Zucker-, ein Phosphorsäure- oder ein Schwefelsäurerest ist, besonders bevorzugt sind dabei Verbindungen, in denen die Carbonsäure, eine $C_1$-$C_{10}$, ganz besonders bevorzugt eine $C_1$-$C_3$, aliphatische Carbonsäure, eine Aminosäure, ganz besonders bevorzugt ein Alanin, das auch derivatisiert sein kann oder eine aromatische Carbonsäure ist.

Besonders bevorzugt sind ferner Verbindungen, worin der Zucker eine α- oder β-Glucose oder eine α- oder β-Galactose ist.

Bevorzugt sind außerdem Verbindungen der Formel I in denen $R^1$ Wasserstoff bedeutet.

Überraschenderweise hat sich gezeigt, daß die erfindungsgemäßen fluorogenen Verbindungen in Abhängigkeit vom gewählten Rest R mit Hydrolasen verschiedener Spezifität in Reaktion treten. Die Anregungs- und Emissionsmaxima der Verbindungen sind um 60 - 90 nm gegenüber denjenigen der zugehörigen freien Fluorophore nach kürzeren Wellenlängen verschoben. Der Stokes-Shift zwischen den jeweiligen Anregungs- und Emissionsmaxima beträgt bis zu 150 nm. Die Herstellung der erfindungsgemäßen Verbindungen erfolgt in an sich bekannter Weise analog literaturbekannten Verfahren aus den Fluoreszenzfarbstoffen (Literatur: Org. Reactions, 6, 1951, Chapter 8 und Heterocyclic Compounds, Vol. 5, 1957, Chapter 6) und dem jeweiligen enzymatisch abbaubaren Rest. So werden fluorogene Substrate für den Nachweis von Phosphatasen und Sulfatasen durch Umsetzen des Fluorophors nach an sich bekannten Verfahren mit geeigneten Säurehalogeniden hergestellt (z. B. Phosphorchloroxid bzw. Chlorsulfonsäure). Entsprechend lassen sich die Carbonester aus den Carbonsäurechloriden herstellen.

Für die Herstellung von Glykosiden werden die Fluoreszenzfarbstoffe nach ebenfalls an sich bekannten Verfahren glykosiliert. Die Herstellung von β-Galactosiden kann beispielsweise durch Umsetzung der entsprechenden Fluoreszenzfarbstoffe mit α-D-Acetobromgalactose und anschließender Entacetylierung erfolgen. Glykosylierungsverfahren sind z. B. beschrieben in Angew. Chemie 98, 1986, 213 - 236 und darin zitierter Literatur. Beispiele für nach den genannten Verfahren erhältliche Glykoside sind z. B. α- und β-D-Galactopyranoside, α-und β-D-Glucopyranoside und sich daraus ableitende Oligosaccharid-Derivate mit 2 - 10, vorzugsweise 3 - 7 Monosaccharideinheiten.

Die erfindungsgemäßen fluorogenen Verbindungen werden für den Nachweis verschiedener hydrolytischer Enzyme, beispielweise Carboxylesterasen, Phosphatasen, Sulfatasen und Glykosidasen, verwendet. Für den Enzymnachweis wird das fluorogene Substrat in einer Reagenzmischung zur Verfügung gestellt, die gegebenenfalls notwendige Puffersubstanzen, Stabilisatoren, Aktivatoren, Lösungsvermittler, Hilfsenzyme oder weitere Hilfschemikalien enthält. Diese Hilfsstoffe sind im einzelnen von den Reaktionsbedingungen, wie z. B. von der Art des zu bestimmenden Enzyms sowie dem Substrat abhängig. Die jeweils zu verwendenden Hilfsstoffe sind dem Fachmann geläufig. Die verschiedenen Einzelchemikalien können bei

hinreichender Stabilität und chemischer Verträglichkeit in einer Lösung nebeneinander vorliegen, sie können aber auch erst kurz vor der Nachweisreaktion miteinander vermischt werden. Der eigentliche Nachweis des hydrolytisch wirksamen Enzyms findet dann durch Messung der Fluoreszenz des durch die enzymkatalysierte Hydrolyse freigesetzten Fluoreszenzfarbstoffs aus dem entsprechenden fluorogenen Substrat nach Zusammenbringen der Reagenzmischung mit dem nachzuweisenden hydrolysierenden Enzym bzw. der zu testenden biologischen Probe statt.

Bevorzugt ist dabei eine Reaktion in Lösung, die gegebenenfalls direkt in einer Küvette durchgeführt werden kann und sofort durch anschließende, fluoreszenzphotometrische Signalermittlung ausgewertet werden kann. Ebenso bevorzugt ist die Applikation der erfindungsgemäßen fluorogenen Substrate auf matrixartige, z. B. fibröse oder filmartige Reagenzienträger, die nach Durchführen der Reaktion eine fluoreszenzphotometrische Signalermittlung gestatten.

Bevorzugt ist ferner die Verwendung der erfindungsgemäßen Verbindungen als Substrat für Enzymimmunoassays, wobei die nachzuweisende Hydrolase an einen Partner eines spezifischen Bindungspaares kovalent gebunden wird, zum Beispiel an einen Antikörper, eine Nucleinsäure, ein Hormon o.ä.. Das Konjugat kann dabei frei oder in an einer Festphase gebundenen Form vorliegen. Festphasen sind beispielsweise partikülare Phasen, bevorzugterweise Latexpartikel oder magnetisierbare Partikel.

Die erfindungsgemäßen fluorogenen Verbindungen können in verschiedener Form zur Verfügung gestellt werden. Bevorzugt sind dabei Ausführungsformen, die bereits eine Kombination der erfindungsgemäßen fluorogenen Substrate mit testnotwendigen Zusatzreagenzien enthalten. Beispiele dafür sind Lösungen, Reagenzientabletten, Pulvergemische oder Lyophilisate, wenn die Nachweisreaktion anschließend in Losung durchgeführt werden soll. Alternativ können die fluorogenen Substrate in Verbindung mit den testnotwendigen Zusatzreagenzien ebenfalls auf saugfähige Träger aufgezogen oder in hydrophile und wasseraufnehmende Filme eingearbeitet werden.

Die Erfindung wird durch folgende Beispiele erläutert.

## Beispiele

### Beispiel 1

Herstellung von 2-Phenyl-6-Hydroxybenzoxazol

0,005 Mol 4-Aminoresorcinolhydrochlorid wurden in 7,5 ml Benzoylchlorid unter Erhitzen gelöst und anschließend 7 Stunden am Rückfluß gekocht. Anschließend wurde das überschüssige Benzoylchlorid im Vakuum abdestilliert, der Rückstand mit 10 ml Äthanol versetzt, kurz aufgekocht und nach Abkühlen mit 10 M NaOH-Lösung alkalisch gestellt. Die alkalische Lösung wurde in 200 ml Wasser eingetragen und am pH-Meter mit konzentrierter Salzsäure auf pH3 eingestellt. Die dabei ausgefallene Kristallmasse wurde abgesaugt und aus 15 ml Toluol umkristallisiert. Das getrocknete Produkt (250 mg) war DC-einheitlich (Kieselgelplatte, Essigester/Eisessig 24 : 1).
$F_P$ = 214° C (Zers)

| Fluoreszenzdaten: | | |
|---|---|---|
| | Excit (nm) | Emiss. (nm) |
| sauer | 308 | 480 |
| basisch | 370 | 480 |

### Beispiel 2

Herstellung von 2-(4′-Trifluormethylphenyl)-6-hydroxybenzoxazol

1,5 ml (10 m Mol) 4-Trifluormethylbenzoesäurechlorid wurden im Ölbad auf 190° C erhitzt und portionsweise mit 1,6 g (10 mMol) 4-Aminoresorcinol versetzt. Nach 1 Stunde wurde das Reaktionsgemisch in 100 ml 10 %ige Sodalösung eingetragen und erwärmt. Das unumgesetzte Säurechlorid wurde dabei verseift. Anschließend wurde das Produkt mit Essigester extrahiert und säulenchromatographisch gereinigt (Kieselgel 60, Essigester oder Chloroform/Eisessig 9 : 1).

| Fluoreszenzdaten: | | |
|---|---|---|
| | Excit (nm) | Emiss. (nm) |
| basisch | 380 | 520 |

Auf analogem Wege waren folgende Produkte erhältlich:

| 2-(4′-Cyanophenyl)-6-hydroxybenzoxazol (CPHB) | | |
|---|---|---|
| basisch | 390 | 540 |
| 2-(4′-Pyridyl)-6-hydroxybenzoxazol | | |
| basisch | 370 | 520 |

Beispiel 3

Herstellung von 2-Cyano-6-hydroxybenzothiazol

Zur Herstellung wird auf Bull. Chem. Soc. Jpn. 36, 332 (1963) verwiesen.
Ausgehend von käuflichen 2-Cyano-6-Methoxybenzothiazol wurde das gewünschte Produkt durch saure Hydrolyse erhalten (Methods in Enzymology, Vol. 13 (1986), S. 20-21).
Ausbeute (500 mg Methoxyverbindung eingesetzt): 130 mg
DC-rein Laufmittel: Chloroform / Essigester 20/5.
$F_p$: 208° C Zers.

| Fluoreszenzdaten: | | |
|---|---|---|
| | Excit (nm) | Emiss. (nm) |
| basisch | 380 | 500 |

Beispiel 4

Glykosylierungsreaktionen

A.) Galactosylierung von 2-(4′-Cyanophenyl)-6-Hydroxybenzoxazol (CPHB)

In einer trockenen Apparatur wurden 0,01 Mol CPHB, 0,01 Mol Acetobromgalactose, 5 m Mol $Ag_2O$ und

0,01 Mol Calziumsulfat x 1/2 $H_2O$ in 100 ml getrocknetem Toluol unter Rückfluß erhitzt. (Als Reaktionsbeschleuniger kann dem Gemisch Chinolin beigefügt werden).
Die Reaktion erfolgte unter Ausschluß von Licht und Wasser (Calziumchloridtrockenrohr auf dem Kühler) unter Rühren.

Nach 2 Stunden Reaktionszeit wurde die Umsetzung dünnschichtchromatographisch kontrolliert. Das entstandene CPHB-acetogalactosid erschien als dunkelblau fluoreszierender Fleck.
Laufmittel: Chloroform/Essigester 4/1 bzw. Chloroform/Aceton 4/1.

Bei nicht ausreichender Umsetzung wurden nochmals Acetobromgalactose und $Ag_20$ zugesetzt und die Mischung wiederum unter Rückfluß erhitzt.
Nach erfolgter Umsetzung wurde die Ansatzlösung filtriert, das Filter mit Toluol gewaschen, Waschtoluol und Mutterlauge vereint und am Rotationsverdampfer bei max. 40° C Badtemperatur bei ca. 70 mbar eingeengt.

Der Rückstand wurde im Vakuumtrockenschrank bei Raumtemperatur und 200 mbar getrocknet. Das verunreinigte Produkt wurde durch Säulenchromatographie gereinigt.
Trennmittel: Kieselgel 60 oder Sephadex RP8.
Laufmittel: Chloroform/Ethylacetat 4 : 1.
Das gereinigte CPHB-tetraacetylgalactosid wurde getrocknet und seine Reinheit dünnschichtchromatographisch überprüft.

B.) Entacetylierung des entstandenen Acetylglykosids

Die Entacetylierung wurde in wasserfreiem Methanol mit Natriummethylat durchgeführt. Dazu wurde das Acetylglykosid (2 mg/dl) in Methanol gelöst und wenig Natriummethylat zugesetzt (5-10 ul einer 30 %igen methanolischen Lösung). Der Verlauf der Entacetylierung wurde mittels Dünnschichtchromatographie überwacht.
Laufmittel: Chloroform/Ethylacetat 4 : 1 und Chloroform/Methanol 3 : 1.

Nach Vollendung der Reaktion wurde die Lösung mit Ionenaustauscher Dowex 50 x 8 neutral bis schwach sauer eingestellt. Die Reinheit wurde über DC geprüft.
Laufmittel: Methanol/Chloroform 1 : 3.
Anschließend wurde die Lösung eingeengt und getrocknet.

C.) Glucosylierung von CPHB

0,01 Mol Brigl's Anhydrid und 0,01 Mol CPHB wurden in 100 ml Toluol unter Rückfluß und Rühren sowie Ausschluß von Wasser erhitzt.
Reaktionszeit: 24 - 48 Stunden

Die Umsetzung wurde dünnschichtchromatographisch überprüft.
Laufmittel: Chloroform/Essigester 4 : 1.

Die Entacetylierung erfolgte wie unter B. beschrieben. Es wurde ein schwach dunkelblau fluoreszierendes $\beta$-Glucosid erhalten.

Beispiel 5

Herstellung des Essigsäureesters

50 mg eines Benzoxazolderivates aus Beispiel 2 wurden in 10 ml THF gelöst und mit 1 ml Acetanhydrid und 100 $\mu$l Pyridin versetzt. Nach 1 Stunde Reaktionszeit wurde das Reaktionsgemisch auf präparativen DC-Platten getrennt.
Laufmittel: Chloroform/Eisessig 9 : 1.

Die Abtrennung zeigte das dunkelblau fluoreszierende Acetat neben unumgesetztem, weißlich fluoreszierendem Ausgangsprodukt. Das Acetat wurde durch Elution der entsprechenden Kieselgelzone gewonnen.

Beispiel 6

Herstellung des Phosphorsäureesters

0,01 Mol 2-Phenyl-6-hydroxybenzoxazol wurden mit 2 ml Diazabicycloundecen in 20 ml Methylenchlorid in Lösung gebracht und auf -4° C abgekühlt. Diese Lösung wurde bei -4° C langsam in eine Mischung aus 20 ml Pyridin und 5 ml Phosphoroxichlorid hineinpipettiert und unter gelegentlichem Umschwenken 2 Stunden bei -4° C stehengelassen. Danach wurde das Reaktionsgemisch in 500 ml 5 % Natriumhydrogencarbonat-Lösung gegeben, 1 Stunde intensiv gerührt und anschließend durch mehrmalige Extraktion mit Essigester von unumgesetztem Ausgangsprodukt befreit. Die wäßrige Lösung wurde auf pH3 gestellt, zum Trocknen eingeengt und das gewünschte Phosphat mit Methanol aus dem Salzgemisch extrahiert. Die weitere Aufreinigung erfolgte chromatographisch (Präparative DC, Laufmittel Essigester/Wasser/Methanol 100 : 25 : 30).

Beispiel 7

Herstellung des N-Toluolsulfonylalaninesters

0,01 Mol Tosylalanin, 1,1 mMol Hydroxybenzothiazol und 1,1 mMol Dicyclohexylcarbodiimid (DCCI) wurden nacheinander in 2 ml THF gelöst und 30 min bei Raumtemperatur gerührt. Danach wurden 0,1 Mol CPHB (Beispiel 2) in 1 ml THF gelöst, die o. g. Lösung zugesetzt und 2 Std. gerührt. Anschließend wurden weitere 2 mMol DCCI nachdosiert und die Reaktionsmischung über Nacht stehengelassen.
Isolation:
Entstandener Dicyclohexylharnstoff wurde durch Zentrifugation entfernt und die klare THF-Lösung in Eiswasser geschüttelt. Die so entstandene wäßrige Phase wurde dreimal mit 50 ml Essigester extrahiert und die vereinigten Extrakte wurden nach Trocknung über Natriumsulfat im Vakuum eingeengt.
Reinigung:
Das Rohprodukt wurde mit präparativer D.C.-Platte (E. Merck) gereinigt.
Laufmittel: Essigester

Beispiel 8

Nachweis von Leukozytenesterasen

Der gemäß Beispiel 7 von der präparativen D.C-.Platte gewonnene methanolische Extrakt unseres gewünschten Produktes wurde auf ein mit einem 0,1 Mol. Borsäure-Hepes-Puffer, pH 7.5, vorgetränkten Filterpapier aufgetragen und getrocknet. Leucozyten im Urin wurden nach Auftropfen auf dieses Indikatorpapier in einer Konzentration von ca. 100 L/µl innerhalb von 15 sec durch Auftreten einer gelben Fluoreszenz angezeigt.

**Ansprüche**

1. Fluorogene Verbindungen der allgemeinen Formel (I)

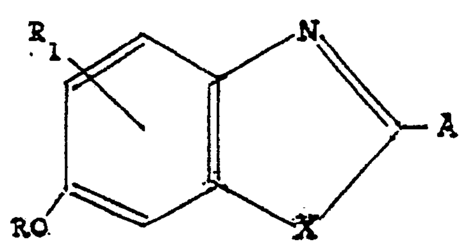

(I)

worin
X 0 oder S bedeutet

R ein durch enzymatisch katalysierte Hydrolyse abspaltbarer Rest ist

$R_1$ H, $C_1$ bis $C_4$ Alkyl oder Phenyl ist, das mit 1 bis 3 $-CH_3$ substituiert sein kann

A eine die Mesomerie des benzoheterocyclischen Ringsystems verlängernde Molokülstruktur aufweist, insbesondere aromatische oder heteroaromatische, auch substituierte und ggf. benzoanellierte 6- und 5-Ringsysteme umfaßt, die auch über eine oder mehrere Mehrfachbindungen in 2-Stellung des Benzoheterocyclus angreifen können, oder eine elektronenziehende Gruppe wie CN oder $CF_3$.

2. Fluorogene Verbindungen gemäß Anspruch 1, wobei

X Sauerstoff bedeutet.

3. Fluorogene Verbindungen gemäß Anspruch 1, worin

A einen Phenyl- oder Naphtylrest bedeutet, der gegebenenfalls durch 1 bis 3 elektronegative Gruppen, wie z. B. -CN, $-CF_3$, Thiazol oder Benzothiazol substituiert ist

oder

einen Thiazol-, Oxazol-, Benzothiazol- oder Benzoxazolrest, der gegebenenfalls mit 1 bis 3 elektronegativen Gruppen, wie z. B. -CN- oder $-CF_3$ substituiert ist, bedeutet.

4. Fluorogene Verbindungen gemäß Anspruch 1, wobei

A Phenyl oder mit bis zu 3 -CN- , $-CF_3$, Thiazol-oder Benzothiazolresten substituiertes Phenyl bedeutet.

5. Fluorogene Verbindungen gemäß Anspruch 1, wobei

R ein Carbonsäure-, ein Zucker-, ein Phosphorsäure-oder ein Schwefelsäurerest ist.

6. Fluorogene Verbindung gemäß Anspruch 5, wobei R eine $C_1$ bis $C_{10}$, bevorzugterweise $C_1$ $-C_3$ aliphatische Carbonsäure, eine Aminosäure, bevorzugterweise ein Alanin oder ein derivatisiertes Alanin, eine aromatische Carbonsäure, eine $\alpha$- oder $\beta$-Glucose oder eine $\alpha$- oder $\beta$-Galactose ist.

7. Fluorogene Verbindungen gemäß Anspruch 1, wobei $R_1$ ein Wasserstoff ist.

8. Verwendung einer fluorogenen Verbindung gemäß Anspruch 1 in einem Test zum quantitativen und/oder qualitativen Nachweis von Hydrolasen.

9. Verwendung gemäß Anspruch 8, wobei die Hydrolase an eine biologisch aktive Substanz, wie z. B. an einen Antikörper, eine Nucleinsäure oder ein Hormon gebunden vorliegt.

10. Verwendung gemäß Anspruch 8, wobei die Hydrolase an eine partikuläre Phase, wie z. B. Latex oder magnetisierbare Partikel, gebunden ist.

11. Reagenzmischung für den Nachweis hydrolysierender Enzyme, die frei oder gebunden vorliegen können, wobei die Reagenzmischung neben dem Fachmann bekannten Hilfsstoffen wie z. B Puffersubstanzen, Stabilisatoren, Aktivatoren, Lösungsvermittlern, Hilfsenzymen, weiteren Hilfschemikalien eine oder mehrere fluorogene Verbindungen nach Anspruch 1 enthält.

12. Reagenzmischung nach Anspruch 11, wobei die fluorogene Verbindung ggf. in Verbindung mit testnotwendigen Zusatzreagenzien in Lösung, als Reagenzientablette, als Pulvergemisch oder Lyophilisat zur Verfügung gestellt wird.

13. Reagenzmischung nach Anspruch 11, wobei die fluorogene Verbindung ggf. in Kombination mit testnotwendigen Zusatzreagenzien auf saugfähige Träger aufgezogen oder in hydrophile Filme eingearbeitet ist.